(19) Europäisches Patentamt
European Patent Office
Office européen des brevets

(11) **EP 1 977 725 B1**

(12) **EUROPÄISCHE PATENTSCHRIFT**

(45) Veröffentlichungstag und Bekanntmachung des Hinweises auf die Patenterteilung:
**24.11.2010 Patentblatt 2010/47**

(51) Int Cl.:
***A61F 9/01*** (2006.01)

(21) Anmeldenummer: **07007119.6**

(22) Anmeldetag: **04.04.2007**

(54) **Vorrichtung für die Materialbearbeitung, insbesondere die refraktive Augenchirurgie**

Device for processing material, in particular refractive eye surgery

Dispositif pour le traitement de matériau, en particulier la chirurgie réfractive de l'oeil

(84) Benannte Vertragsstaaten:
**DE ES FR GB IT**

(43) Veröffentlichungstag der Anmeldung:
**08.10.2008 Patentblatt 2008/41**

(73) Patentinhaber: **WaveLight GmbH**
**91058 Erlangen (DE)**

(72) Erfinder:
• **Vogler, Klaus, Dr.**
**90542 Eckental (DE)**

• **Donitzky, Christof**
**90542 Eckental (DE)**

(74) Vertreter: **von Hellfeld, Axel**
**Wuesthoff & Wuesthoff**
**Patent- und Rechtsanwälte**
**Schweigerstrasse 2**
**81541 München (DE)**

(56) Entgegenhaltungen:
**WO-A-03/011175      US-A1- 2006 095 023**

**Beschreibung**

Die Erfindung betrifft eine Vorrichtung für die Materialbearbeitung, insbesondere die refraktive Augenchirurgie mit einer gepulsten Laserstrahlquelle, Mitteln zum Fokussieren und Führen des von der Laserstrahlquelle emittierten Laserstrahls auf zu bearbeitendes Material, wie insbesondere ein Auge, und mit einer rechnergestützten Steuerung zum Steuern der Führungsmittel derart, dass die Fokusse des Laserstrahls auf einer gesteuerten Bahn geführt werden.

Nachfolgend wird die Erfindung mit Blick auf die refraktive Augenchirurgie, insbesondere mit Blick auf die LASIK beschrieben.

Die LASIK ist ein inzwischen weitgehend etabliertes Verfahren der refraktiven Chirurgie. Bei der refraktiven Chirurgie werden die Brechungseigenschaften des Auges mit Laserstrahlung geändert.

Ein bei der LASIK zunehmend an Bedeutung gewinnendes Instrument ist der Femtosekundenlaser, also ein gepulster Laser mit extrem kurzen Pulslängen, die im Bereich bis zu einigen hundert Femtosekunden liegen können. Aufgrund dieser kurzen Pulslängen ist es möglich, durch Fokussierung der Strahlung innerhalb sehr kleiner Volumina extrem hohe Leistungsdichten elektromagnetischer Strahlung und damit extrem hohe Feldstärken zu erzeugen. Der Femtosekundenlaser dient in der LASIK zur Zeit hauptsächlich als ein Instrument zum Erzeugen des sogenannten Flap-Schnittes, also eines Schnittes durch die Kornea zur Erzeugung eines Deckelchens ("Flap"), das in der Regel über ein kleines Randstück mit der Kornea verbunden bleibt, sodass es aufgeklappt werden kann, um darunterliegendes Stroma freizulegen, welches dann mit einem (anderen) Laserstrahl gemäß einem zuvor berechneten Ablationsprofil abgetragen wird. Nach dieser Neuformung der Kornea wird das Deckelchen zurückgeklappt und verheilt in der Regel sehr schnell wieder mit der Kornea. Der Femtosekundenlaser löst mehr und mehr das Mikrokeratom ab. Das Mikrokeratom ist eine mechanische Vorrichtung mit einer oszillierenden Klinge, mit der der vorstehend beschriebene Schnitt zur Erzeugung des Deckelchens ebenfalls durchführbar ist.

Man schätzt, dass bis zur Zeit mehr als 1 Millionen Operationen dieser Art weltweit mit einem Femtosekundenlaser durchgeführt worden sind.

Der Einsatz des Femtosekundenlasers für den vorstehend beschriebenen Schnitt in der Kornea wird auch als Fs-LASIK bezeichnet. Die Fs-LASIK hat gegenüber dem Einsatz eines mechanischen Mikrokeratoms eine Reihe von Vorteilen, wie zum Beispiel ein geringeres Komplikationsrisiko, eine höhere Genauigkeit der gewünschten Schnittdicke des Flaps und auch einen besser ausgeformten Randschnitt.

Um allerdings mit einem Fs-Laser eine Qualität des Schnittbettes zu erreichen wie mit der präzisen Klinge eines Mikrokeratoms und auch um eine komplikationsfreie Ablösung des Flaps nach dem Schnitt zu ermöglichen, müssen bei der Fs-LASIK die Parameter des Verfahrens sehr fein optimiert werden, insbesondere die Schnittparameter (siehe unten).

Die Ursache für diese erforderliche Feinoptimierung der Fs-LASIK liegt in der Physik der Erzeugung des Schnittes. Grundsätzlich entsteht der Fs-LASIK-Schnitt durch eine dichte Aneinanderreihung von kleinen sogenannten Mikrodissektionen mit zum Beispiel einem Durchmesser im Bereich von 5 $\mu$m. Das Gewebe wird durch die extrem hohe lokale Leistungsdichte der Strahlung (d.h. die hohe Feldstärke) aufgebrochen und es erfolgt eine lokale Durchtrennung des Korneagewebes und der darin eingelagerten Mikrofibrillen. Durch die Gesamtheit der eng benachbarten fokussierten Pulse entsteht letztlich eine flächenhafte Durchtrennung des Gewebes. Die erforderlichen Feldstärken werden mit heute verfügbaren Lasern in der Regel nur im Fokus erreicht. Dies hat wiederum den Vorteil, dass die Gewebedurchtrennung auch in der Tiefe unterhalb der Gewebeoberfläche genau an der Stelle des Brennpunktes bewirkt werden kann.

Die oben genannten, sehr empfindlich zu optimierenden Verfahrensparameter sind insbesondere die Laserpulsenergie, der Fokuspunktdurchmesser, der Fokuspunktabstand, sowie die Steuerung der einzelnen fokussierten Pulse in Zeit und Raum.

Für die Durchführung des Fs-LASIK-Schnittes mit einer Aneinanderreihung von dicht benachbarten Mikrodissektionen durch enge Platzierung und zeitliche Abfolge von auf einer Bahn geführten Fokuspunkten gibt es im Stand der Technik verschiedene Ansätze. Ein Kriterium dabei ist auch der Zeitbedarf für die Durchführung des Gesamtschnittes zur Erzeugung des Flaps.

Der Stand der Technik kennt zum Beispiel die Führung der Brennpunkte der einzelnen Strahlungspulse von Puls zu Puls entlang einer spiralförmigen Bahn und insbesondere auch eine zeilenförmige Führung der zeitlich aufeinanderfolgenden Brennpunkte, ähnlich etwa der Steuerung des Elektronenstrahls bei einer herkömmlichen Bildröhre.

Die Mittel, mit denen die Laserstrahlung für die vorstehend beschriebenen Zwecke geformt und im Raum geführt wird, sind im Stand der Technik bekannt. Die vorstehend genannte zeilenförmige Rasterung der Brennpunkte ist weit verbreitet, weil dabei auf verfügbare Abtasttechniken (Spiegel und deren Steuerungen) zurückgegriffen werden kann. Um mit einer solchen zeilenweisen Führung der Brennpunkte aufeinanderfolgender Laserstrahlungspulse einen guten Fs-LASIK-Schnitt zu erzeugen, können zum Beispiel folgende Verfahrensparameter geeignet sein:

-   Laserpulsenergie:          1 $\mu$J
-   Fokusdurchmesser:         < 5 $\mu$m

- Fokusabstand in der Zeile: $\sim 8~\mu m$
- Zeilenabstand: $\sim 12~\mu m$
- Durchmesser des Flaps: 9 mm
- Laserpulsfolgefrequenz: 60 kHz

Mit diesen Parametern lässt sich zum Beispiel ein Flapschnitt in weniger als 30 Sekunden erreichen.

Allerdings hat sich in jüngerer Zeit im Zusammenhang mit Fs-LASIK-Schnitten ein Phänomen ergeben, das derart behandelte Patienten irritiert. Patienten sehen nach einer Fs-LASIK-Operation bisweilen an scharfen Kanten von Objekten farblich aufgelöste Randstrukturen, also eine Art Regenbogen. Dies wird als "Rainbow-Glare"-Effekt bezeichnet.

Die WO 03/011175 diskutiert eine irreguläre Abfolge von Laser-Ablationsimpulsen, um die Ortsfrequenz des Abtrags niedrig zu halten und gegenseitige thermische Beeinträchtigungen der Abtragsbereiche bei der PRK zu vermeiden und so eine geringe Oberflächenrauheit zu erreichen.

Der US 2006/0095023 liegt die Überlegung zugrunde, dass bei der Materialbearbeitung mit fokussierten Laserpulsen, zum Beispiel in der Kornea, die entstehenden Effekte ein bestimmtes Zeitverhalten haben. Die Wirkung des einzelnen Laserpulses besteht in der Erzeugung von Kavitäten, die sich aufblähen und wieder "zusammenfallen". Um zu vermeiden, dass ein in der Pulssequenz sich an einen vorangehenden Puls anschließender Puls genau in den Bereich der Wirkung des vorangegangenen Pulses fällt, wird der Abstand eines nachfolgenden Pulses so groß gemacht, dass er außerhalb der zuvor entstandenen Kavität liegt.

Der Erfindung liegt die Aufgabe zugrunde, einen solchen Regenbogeneffekt zu vermeiden.

Die Aufgabe der Erfindung wird mittels einer Vorrichtung nach Anspruch 1 gelöst.

Die Erfindung geht von der Annahme aus, dass die genannten Regenbogeneffekte dadurch zustande kommen, dass bei herkömmlicher Steuerung der räumlichen Positionen der Strahlungsbrennpunkte Strukturen in der Kornea erzeugt werden, die hernach physikalisch wie zum Beispiel ein Gitter wirken, welches durchtretendes weißes Licht durch Beugung in seine spektralen Anteile zerlegt. Mit anderen Worten: Beim Stand der Technik entstehen durch die dort gewählte räumliche Positionierung der einzelnen Strahlungsbrennpunkte regelmäßige Strukturen mit äquidistanten Fokusabständen, die insbesondere ein zweidimensionales Gitter bilden können, welches im Auge auf der Retina Beugungsbilder erzeugt, sodass die einzelnen Farben des weißen Lichtes bei Betrachtung einer scharfen Kante nicht mehr genau übereinanderfallen.

Es versteht sich, dass dieser Effekt in der refraktiven Chirurgie höchst unerwünscht ist.

Gemäß der Erfindung werden nun die Abstände benachbarter Fokusorte so gewählt, dass die genannten regelmäßigen Strukturen in dem bearbeiteten Material, insbesondere in einer Kornea, nicht mehr auftreten. Mit anderen Worten: Gemäß der Erfindung werden die einzelnen Wirkungspunkte der Laserstrahlung so positioniert, dass keine regelmäßigen, unerwünschte Beugungserscheinungen hervorrufenden Strukturen mehr entstehen.

Im Falle des Fs-LASIK-Schnittes herkömmlicher Art entstanden solche, den unerwünschten Beugungseffekt verursachenden regelmäßigen Strukturen dadurch, dass die einzelnen Fokusse der Laserstrahlung im wesentlichen äquidistant positioniert worden sind und so auch nach zurückgeklapptem Flap und vollendetem Heilungsprozess regelmäßige Gitterstrukturen mit lokaler Variation des Brechungsindex verblieben.

Gemäß einer bevorzugten Ausgestaltung der Erfindung werden die Abstände benachbarter Fokuslagen stochastisch variiert. Dabei kann zum Beispiel für die Berechnung der einzelnen Positionen von räumlich aufeinanderfolgenden Fokussen ein bei der Rechnung gleichbleibender Grundabstand vorgegeben werden, der dann von Puls zu Puls innerhalb vorgegebener geringer Grenzen variiert wird. Dabei sind die vorgegebenen Abweichungsgrenzen (also die Grenzen der Abweichung gegenüber eine äquidistanten Brennpunktfolge) so gewählt, dass die Brennpunkte trotz ihrer ungleichmäßigen Abstände im Ergebnis einen sauberen Schnitt erzeugen. Zum Beispiel können die Abweichungsgrenzen auf 5 bis 20 % der Konstanten des genannten rechnerischen Grundgitters eingestellt werden, wobei die Gitterkonstante des rechnerischen Grundgitters klein genug ist, um auch bei größtmöglichem Abstand der so erzeugten Fokuslagen ununterbrochene Mikrodissektionen zu bewirken.

Nachfolgend wird ein Ausführungsbeispiel der Erfindung anhand der Zeichnung näher beschrieben. Es zeigt:

Figur 1   schematisch eine Vorrichtung für die refraktive Augenchirurgie;

Figur 2   ein Ausführungsbeispiel für eine Bahn, gemäß der Fokusse von Fs-Laserpulsen in einer Kornea in zeitlicher Abfolge in einer Kornea gesteuert werden; und

Figur 3   einen Ausschnitt aus Figur 2, wobei die regelmäßige Äquidistanz zwischen einzelnen Fokuslagen aufgehoben ist.

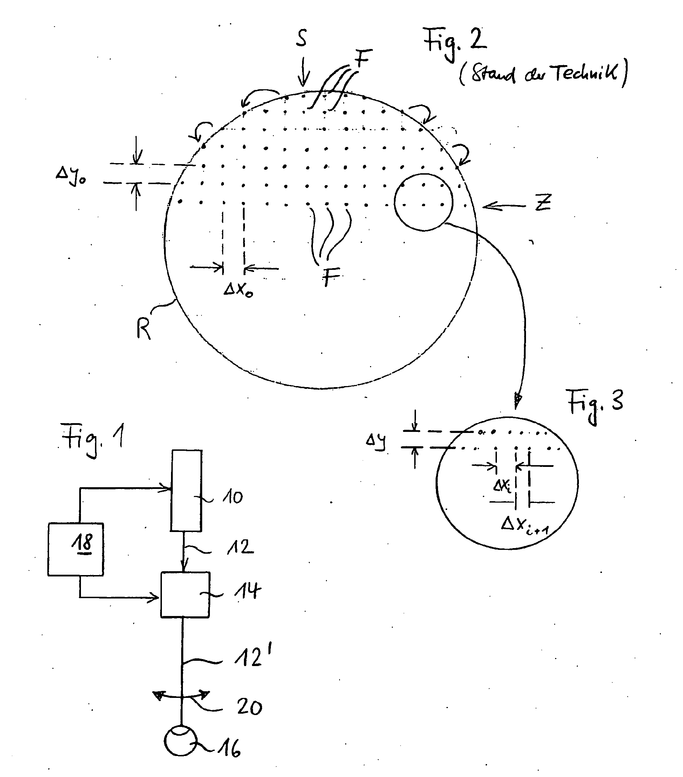

Figur 1 zeigt schematisch eine als solche bekannte Vorrichtung für die refraktive Augenchirurgie mit einer Laserstrahlquelle 10 zur Erzeugung von Laserpulsen mit Pulslängen im Femtosekundenbereich, wobei die emittierten Pulse mit dem Bezugszeichen 12 angedeutet sind. Die Laserpulse werden in Mittel 14 gerichtet zum Formen, insbesondere Fokussieren, und Führen der Laserstrahlpulse 12' in Richtung auf ein Auge 16. Die Mittel zum Formen, Fokussieren und Führen der Strahlung sind als solche ebenfalls bekannt. Eine rechnergestützte Steuerung 18 steuert die Laserstrahlquelle und die Mittel 14 zur Strahlformung und -führung. Zum Beispiel werden die Laserstrahlpulse 12' entsprechend dem Pfeil 20 über das refraktiv zu behandelnde Auge geführt. Da es sich um diskrete Strahlungspulse handelt, kann diese Führung über das Auge auch als "Rasterung" bezeichnet werden.

Figur 2 zeigt schematisch eine solche Rasterung der Brennpunkte F bei einem Fs-LASIK-Schnitt. Der Rand des Schnittes ist mit R bezeichnet. Zum Beispiel kann der Schnitt einen Durchmesser von 9 mm haben. In Figur 2 sind nur in der oberen Hälfte die einzelnen Fokusse F dargestellt, die untere Hälfte der Fokusse ist entsprechend ergänzt zu denken.

Die Fokusse F der zeitlich sequentiellen Laserpulse 12' werden in Zeilen Z geführt, wobei die Zeilensprünge durch Pfeile markiert sind. Die zeitliche Abfolge der Pulse verläuft also linear in Zeilen von rechts nach links bzw. links nach rechts. Es entsteht, wie Figur 2 zeigt, eine regelmäßige Gitterstruktur, bei der benachbarte Fokuspunkte F in einer Zeile Z immer den gleichen Abstand $\Delta x_0$ haben. Auch der Abstand $\Delta y_0$ ist zwischen den Zeilen konstant. Diese regelmäßige Gitterstruktur gilt es, wie oben ausgeführt ist, zu vermeiden.

Hierzu werden bei dem dargestellten Ausführungsbeispiel gemäß Figur 3 in einer Zeile Z die Abstände $\Delta x$ zwischen benachbarten Fokuslagen variiert. Zum Beispiel ist der dargestellte Abstand $\Delta x_i$ zweier benachbarter Fokusse F größer als der Abstand $\Delta x_{i+1}$ im nachfolgenden Intervall zwischen zwei Brennpunkten.

Diese Variation von Abständen erfolgt unregelmäßig über jede der Zeilen bei zumindest einem Großteil der Fokusabstände. Die Maßgabe "zumindest bei einem Großteil" ist so zu wählen, dass insgesamt keine hinreichend regelmäßige Gitterstruktur entsteht, die störende Beugungseffekte im oben beschriebenen Sinne erzeugen kann. Unter dieser Vorgabe stören einige wenige äquidistante Fokuslagen nicht.

Eine Möglichkeit, den Fokusabstand $\Delta x$ innerhalb einer Zeile zu variieren ist folgender stochastischer Ansatz:

$$\Delta x_i = \Delta x_0 + a\,(i \cdot \Delta x_0)$$

zwischen dem (i-1)-ten Fokus und dem i-ten Fokus

$$\Delta x_{i+1} = \Delta x_0 + a\,[(i+1) \cdot \Delta x_0]$$

zwischen dem i-ten und (i+1)-ten Fokus,

z.B. $\quad \Delta x_0 = \text{const} = 5\ \mu\text{m}$

$a = 0{,}10$       prozentuale Modulationsrate

$I;(I+1)$       generierte Zufallszahlen zwischen 0 ... 1

$\Delta x_0$ ist der rein rechnerische vorgegebene Grundabstand benachbarter Foki (Brennpunkte) in einer Zeile Z. Dieser Abstand wird gemäß den vorstehenden Formeln stochastisch, also gemäß einer Zufallsfolge, in Grenzen variiert. Zum Beispiel beträgt der rechnerische Grundabstand $\Delta x_0$ 5 $\mu$m bei einem Fokusdurchmesser von 3 $\mu$m. Der Faktor a gibt die Grenzen für die zulässige Variation des Abstandes benachbarter Foki an. Beträgt a = 0,10, dann liegt die zulässige Variation der Fokusabstände bei 10%. Der Faktor a bestimmt also den Modulationshub der Fokusabstände. Die Werte i, (i+1) sind mit einem Zufallsgenerator generierte Zufallszahlen im Zahlenintervall [0 .... 1], die für den Einzelfall des Abstandes benachbarter Foki stochastisch bestimmen. Die Parameter $\Delta x_0$, a, i werden so gewählt dass trotz der Abstandsvariationen die Brennpunkte hinreichend eng beieinanderliegen, um ein gut zusammenhängendes, "kontinuierliches" Schnittbett zu erzeugen.

Entscheidend ist, dass die Variation der Fokusabstände zur Folge hat, dass kein für die jeweilige Anwendung störendes Beugungsbild mehr durch eine regelmäßige Gitterstruktur entstehen kann.

Analog könnte auch, entweder für sich oder zusätzlich zur Variation der Abstände in einer Zeile, auch der Fokusabstand in den Spalten S variiert werden. Entsprechend könnte also die Regelmäßigkeit der Fokusabstände in y-Richtung wie folgt aufgehoben werden:

$$\Delta y_i = \Delta y_0 + b\,(i \cdot \Delta y_0)$$

Linienabstand zwischen der (i-1)-ten Zeile und i-ten Zeile

$$\Delta y_{i+1} = \Delta y_0 + b\,[(i+1) \cdot \Delta y_0]$$

Linienabstand zwischen i-ten und (i+1)-ten Zeile,

(fortgesetzt)

| z.B. | b=0,15 | Modulationsbreite |
| | $\Delta y_0 = 10\ \mu m$ | Zeilenabstand |

Dabei haben die einzelnen Parameter analoge Bedeutungen wie oben anhand der Abstandsvariationen in der Zeile Z beschrieben ist. Der Faktor b = 0,15 gibt also die Grenzen für die Abstandsvariation in y-Richtung an, hier sind es 15% und $\Delta y_0$ ist der rechnerisch vorgegebene Grundabstand von Brennpunkten in der Spalte S. Auch hier müssen die einzelnen Parameter so gewählt und optimiert werden, dass in jedem Falle bei dem vorgegebenen Fokusdurchmesser und den eingestellten Pulsenergien des Fs-Lasers sich eine gut zusammenhängende Schnittfläche ergibt.

Insgesamt entsteht dann im Schnittbett eine verbleibende Restrauhigkeit mit Dichteschwankungen, die aber hinreichend irregulär sind, um unerwünschte Beugungseffekte auszuschließen.

Theoretisch ist denkbar, den vorstehend beschriebenen Effekt variierender Fokusabstände, die rechnerisch über einen Zufallsgenerator oder dergleichen gewonnen werden, auch durch eine mechanisch instabile ("vibrierende") Strahlführung zumindest teilweise zu erreichen, jedoch ist eine rechnerische Steuerung und Beherrschung des Prozesses vorzuziehen.

**Patentansprüche**

1. LASIK-Flap-Schnitt-Vorrichtung für die refraktive Augenchirurgie mit

   - einer gepulsten Laserstrahlquelle (10),
   - Mitteln (14) zum Fokussieren und Führen des von der Laserstrahlquelle emittierten Laserstrahls (12) auf ein Auge (16),
   - einer rechnergestützten Steuerung (18) zum Steuern der Führungsmittel (14) derart, dass die Fokusse (F) des geführten Laserstrahls (12') auf einer vorgegebenen Bahn (S, Z) am oder im Auge geführt werden, wobei
   - die Bahn zeilenförmig oder spaltenförmig ist,

   **dadurch gekennzeichnet, dass**

   - die Abstände ($\Delta x_i$) von in einer Zeile (Z) benachbarten Fokussen (F) stochastisch variieren oder
   - die Abstände ($\Delta y_i$) von in einer Spalte (S) benachbarten Fokussen (F) stochastisch variieren.

2. LASIK-Flap-Schnitt-Vorrichtung nach Anspruch 1, **dadurch gekennzeichnet, dass** die Laserstrahlquelle (10) ein Femtosekundenlaser ist.

**Claims**

1. Apparatus for cutting a flap in a LASIK procedure for refractive eye surgery having

   - a pulsed laser beam source (10),
   - means (14) for focussing and guiding the laser beam (12) emitted by the laser beam source onto an eye (16),
   - a computer assisted controller (18) for controlling the guide means (14) so that the foci (F) of the guided laser beam (12') are guided on a predetermined path (S, Z) on or in the eye,

   wherein

   - the path is linewise or columnwise,

   **characterized in that**

   - the spacings ($\Delta x_i$) of neighbouring foci (F) in a line (Z) vary stochastically, or
   - the spacings ($\Delta y_i$) of neighbouring foci (F) in a column (S) vary stochastically.

2. Apparatus for cutting a flap in a LASIK procedure in accordance with claim 1, **characterized in that** the laser beam source (10) is a femtosecond laser.

**Revendications**

1.  Dispositif de découpe du capot LASIK pour la chirurgie réfractive, comprenant

    - une source de rayonnement laser pulsé (10),
    - des moyens (14) pour focaliser et diriger sur un oeil (16) le rayon laser (12) émis par ladite source de rayonnement laser,
    - une commande (18) assistée par ordinateur pour commander de telle sorte les moyens de guidage (14) que les points focaux (F) du rayon laser guidé (12') sont dirigés le long d'une trajectoire prédéfinie (S, Z) ou dans l'oeil,

    la trajectoire étant de forme linéaire ou colonnaire,
    **caractérisé en ce que**

    - les distances ($\Delta x_i$) séparant les points focaux (F) adjacents à l'intérieur d'une ligne (Z) varient de manière stochastique
    - les distances ($\Delta y_i$) séparant les points focaux (F) adjacents à l'intérieur d'une colonne (S) varient de manière stochastique.

2.  Dispositif de découpe du capot LASIK selon la revendication 1, **caractérisé en ce que** la source de rayonnement laser (10) est un laser femtoseconde.

Fig. 2

(Stand der Technik)

S

F

$\Delta y_0$

Z

$\Delta x_0$

F

R

Fig. 3

$\Delta y$

$\Delta x_i$

$\Delta x_{i+1}$

Fig. 1

18

10

12

14

12'

20

16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 03011175 A
- US 20060095023 A